# EUROPÄISCHE PATENTANMELDUNG

(11) **EP 1 332 754 A1**
(43) Veröffentlichungstag der Anmeldung: **06.08.2003**
(21) Anmeldenummer: 02405060.1
(22) Anmeldetag: 31.01.2002
(51) Int. Cl.: A61K 9/00, A61F 9/00, A61K 45/06

(54) **Material abgebender Träger**

(71) Anmelder: Mojon, Daniel, 9008 St. Gallen (CH)
(72) Erfinder: Mojon, Daniel, 9008 St. Gallen (CH)
(74) Vertreter: Roshardt, Werner Alfred, Dipl.-Phys.

(57) **Zusammenfassung**

Ein Material abgebender Träger weist wenigstens einen Bereich für ein erstes Material **(7)** und wenigstens einen zweiten vom ersten getrennten Bereich für ein zweites Material **(9)** auf. Aufgabe der Erfindung ist es, z. B. einerseits das zeitlich aufwendige und umständliche Einfärben des Auges zu erleichtern und zudem auszuschliessen, dass beide Materialien miteinander reagieren können, um eine lange Haltbarkeit zu gewährleisten. Die Erfindung ist nicht auf einen Einsatz in der Ophthalmologie beschränkt. Der Material abgebende Träger kann überall dort verwendet werden, wo mehrere Materialien zusammen vorzugsweise an schwer zugängliche Stellen oder mit grosser Sorgfalt gebracht werden müssen.

## Beschreibung

### Technisches Gebiet

Die Erfindung betrifft einen Material abgebenden Träger gemäss dem Oberbegriff des Patentanspruchs 1 sowie eine Trägeranordnung mit mehreren Trägern.

### Stand der Technik

Zur Behandlung und/oder Diagnose am menschlichen bzw. tierischen Körper, zum Einbringen in Körperöffnungen, in das oder auf das Auge (beispielsweise ins Augenlid bei der Tonometrie) müssen oftmals mehrere Flüssigkeiten an anähernd einund demselben Ort gebracht werden.

Bei einer Tonometrie z. B. wird auf das Auge erst ein Anästhetikum und dann Fluorescein gegeben, um überhaupt erst den Abplattungsgrad bei einem Goldmann-Tonometer bestimmen zu können. Für diese Messung träufelt der Augenarzt dem Patienten erst einen Tropfen Anästhetikum aus einer Tropfflasche unter genauem Zielen ins Auge. Anschliessend wird ein mit Fluorescein getränkter Filtersteifen ins Augenlid eingetupft, wodurch die Tränenflüssigkeit Fluorescein herauslöst und über das Auge verteilt.

Dieses Verfahren ist für den Augenarzt zeitaufwendig und umständlich zu handhaben, da das Eintropfen meistens durch die vor dem Patientengesicht sich befindenden Aufbauten eines Spaltlampengeräts räumlich behindert wird. Der Augenarzt muss aus seiner Beobachtungsstellung in der Regel am Spaltlampengerät aufstehen und über das Gerät greifen.

Die US-A 3 374 144 schlägt nun zur Vereinfachung der Augendruckmessung vor, Fluorescein mit einem passenden Anästhetikum zu mischen und beides als Mischung auf das Auge bzw. in das Augenlid zu träufeln.

### Darstellung der Erfindung

### Aufgabe der Erfindung

Aufgabe der Erfindung ist es, insbesondere das zeitlich aufwendige und umständliche Einfärben des Auges zu erleichtern und zudem auszuschliessen, dass beide Materialien in einem Gebinde miteinander reagieren können, um eine lange Haltbarkeit zu gewährleisten.

### Lösung der Aufgabe

Die Aufgabe wird dadurch gelöst, dass beide Materialien, Fluorescein und Anästhetikum, auf einem Träger voneinander getrennt vorhanden sind. Die beiden Materialien, fest oder flüssig, können sich nun während der Lagerung nicht mehr mischen und dadurch auch nicht mehr gegenseitig beeinflussen; eine hierdurch mögliche Beeinträchtigung der Haltbarkeit ist aufgehoben. Auch kann kein chemisches Einwirken des einen Materials auf das andere erfolgen.

Werden, wie gerade angetönt, die beiden Materialien Fluorescein und ein Anästheitkum auf einem Träger angeordnet, bietet sich eine Verwendung bei der Bestimmung des Augeninnendruckes mit einem Goldmann-Tonometer an.

Der erfindungsgemässe Träger ist jedoch nicht auf eine Verwendung in der Ophthalmologie beschränkt; es können auf ihm auch andere, z.B. technisch einsetzbare Materialien voneinander getrennt angeordnet werden.

Wird der Träger stabförmig ausgebildet und die wenigstens beiden Materialien voneinander getrennt in einem Stabendbereich angeordnet, so lässt er sich bevorzugt überall dort verwenden, wo Körperöffnung (Nasenloch, Ohrengang, ...) oder Körperzwischenräume (hinter dem Ohrläppchen, zwischen den Zehen, zwischen den Fingern, ...) zu behandeln sind bzw. die Materialien als Diagnosehilfsmittel dienen.

Neben einem Einsatz am menschlichen bzw. tierischen Körper kann ein derartiger Träger auch zum technischen Einsatz (Bohrlochrand, Schlitzrand, Ecken, ...) kommen.

Der Träger kann nun als Stab mit beliebigem Querschnitt, rund, oval oder als Vierkantstab ausgebildet sein. Er kann aber auch aus einem Filterpapierstreifen bestehen. Kunststoffstäbe sind selbstverständlich auch möglich.

Bei einem Einsatz in der Ophthalmologie wird man das Fluorescein in wasserlöslicher Form aufbringen und eintrocknen lassen. Für das Aufbringen und Anordnen des Anästhetikums sind mehrere Verfahren möglich, je nachdem welche Art von Anästhetikum verwendet wird. Werden wasserlösliche Anästhetika verwendet, welche ihre Wirkung auch beim Eintrocknen nicht verlieren, kann mit ihnen analog zum Fluorescein verfahren werden.

Damit die beiden flüssigen Materialien nicht ineinander verlaufen, kann zwischen ihnen eine Flüssigkeitssperre vorgesehen werden. Mannigfaltige Flüssigkeitssperren sind möglich. Es ist lediglich darauf zu achten, dass der als Flüssigkeitssperre dienende Zwischenraum bei nebeneinander liegenden Materialien eine ausreichende Zwischenraumbreite und eine ausreichende Flieshemmung für die Materialien hat, damit deren Zusammenfliessen als Flüssigkeit während des Aufbringens und einer anschliessenden Trocknungsphase vermieden bzw. stark unterbunden wird. Bei auf der Vorder- und der Rückseite liegenden Materialien muss das Stäbchenmaterial und dessen Dicke bzw. Konsistenz entsprechend beschaffen sein.

Man kann das Anästhetikum jedoch auch in flüssiger Form in einem Kissen auf dem Träger einschliessen. Das Kissen wird man dann bevorzugt mit wenigstens einer Aufreisslinie versehen, welche durch Druck, vorzugsweise durch Fingerdruck, aufreisst, und bei einem Anliegen an das Augenlid das Anästhetikum in dieses übertreten lässt. Je nachdem wie das Aufreissen erfolgen soll, können auch mehrere Aufreisslinien vorhanden sein.

Die Träger wird man vorzugsweise einzeln steril verpacken und die Verpackung mit einer Aufreisseinrichtung z. B. einem Aufreissfaden versehen.

Anstatt die Träger einzeln zu verpacken und auszubilden, können auch mehrere Träger zusammen in der Art eines "Zündholzbriefchens" angeordnet werden. Dieses "Zündholzbriefchen" kann nun als Ganzes steril verpackt werden. Es können aber auch hier die einzelnen Stäbchen als Träger, welche an ihren "Fussenden" über eine Abreisslinie zusammenhängen, steril verpackt sein. Die sterile Einzelverpackung der Stäbchen im Briefchen ist dann ebenfalls kissenförmig mit jeweils einer Trennlinie zwischen den Stäbchen ausgebildet.

Die oben erwähnten Träger, sofern sie zur Augeninnendruckmessung verwendet werden, tragen Fluorescein und ein Anästhetikum. Für andere Verwendungen wird man andere Materialien benützen. Die Erfindung ist jedoch nicht auf die Verwendung von nur zwei Materialien beschränkt; es können selbstverständlich auch mehrere Materialien voneinander getrennt aufgebracht werden.

Werden nur zwei Materialien verwendet, kann man sie bei einem flachen Stab auf der Vorder- und der Rückseite am Stabende aufbringen, wobei dann die Materialien durch den Stab voneinander getrennt sind. Die Materialien können jedoch auch nebeneinander aufgebracht werden.

### Kurze Beschreibung der Zeichnungen

Im Folgenden werden Beispiele des erfindungsgemässen Trägers anhand der nachfolgenden Zeichnungen näher erläutert. Weitere Vorteile der Erfindung ergeben sich aus dem Beschreibungstext. Es zeigen:
- Fig. 1: eine perspektivische Darstellung eines Trägers für zwei Materialien;
- Fig. 2: eine Vergrösserung der Materialanordnung des in **Figur 1** dargestellten Trägers,
- Fig. 3: eine Variante zur Ausführung in **Figur 1**, wobei hier die Materialien nebeneinander angeordnet sind, und
- Fig. 4: eine Anordnung mehrerer Träger.

### Wege zur Ausführung der Erfindung

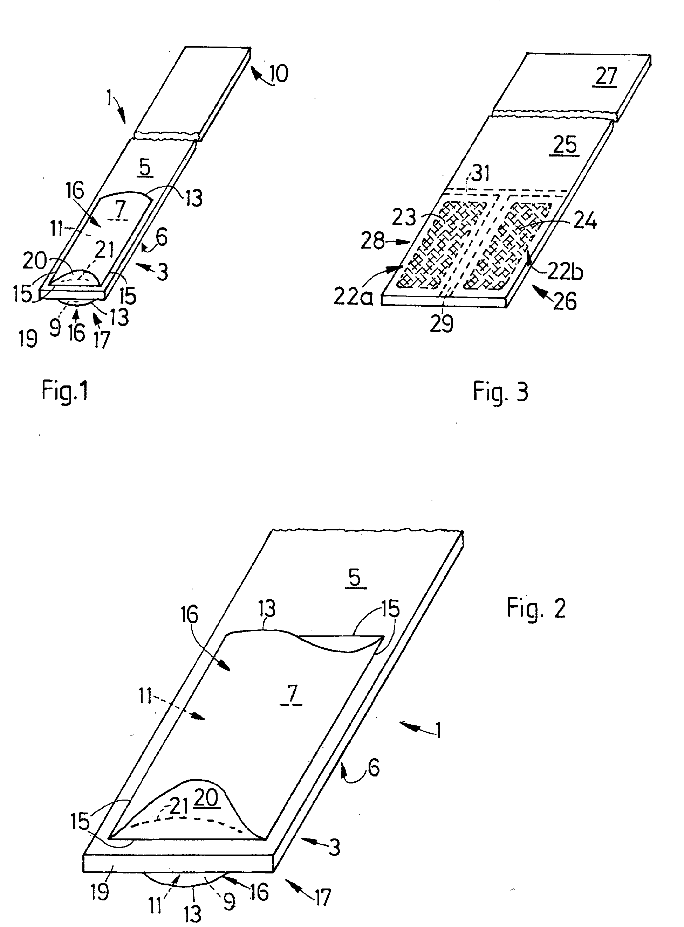

Der unten beschriebene, erfindungsgemässe, Material abgebende Träger (**Fig. 1**) hat ein Stäbchen **1** mit einem quaderförmigen Querschnitt, an dessen einem Endbereich **3** auf der einen Oberseite **5** ein erstes und auf der anderen Oberseite **6** ein zweites Material **7** bzw. **9** aufgebracht ist. Das Stäbchen **1** besteht je nach aufzubringendem Material aus einem Streifen Filterpapier, steifen, aber dennoch leicht biegsamen Karton oder Kunststoff. Die Abmessungen des Stäbchens **1** richten sich nach der Verwendung. Wird der Träger in der Ophthalmologie bei der Augeninnendruckmessung (Tonometrie) verwendet, ist beispielsweise das erste Material **7** Fluorescein und das zweite Material **9** ein Anästhetikum. Die geometrischen Abmessungen des Stäbchens wird man dann derart wählen, dass es an dem dem Endbereich 3 abgewandten Endbereich **10** gut zwischen Zeigefinger und Daumen gehalten werden kann. D.h. bei dieser speziellen Anwendung beträgt die Breite des Stäbchens einige Millimeter und die Länge einige Zentimeter.

Im Gegensatz zur Darstellung in **Figur 1** muss das Stäbchen **1** nicht über seine gesamte Länge gleich breit und gleich dick sein. Die Breite im Endbereich **10** wird man immer auf eine gute Ergreifbarkeit auslegen. Die Breite im Endbereich **3** wird man jedoch den aufzubringenden Materialien, dessen Volumen und der speziellen Anwendung anpassen. D.h. die Breite kann hier grösser oder kleiner als im Endbereich **10** sein. Auch kann eine unterschiedliche Dicke gewählt werden.

Die Speicherung der beiden Materialien **7** und **9** auf den beiden Oberseiten **5** und **6** des Endbereichs **3** richtet sich ebenfalls nach dem Einsatzgebiet und den verwendeten Materialien bzw. deren Möglichkeit für eine Langzeitspeicherung.

Werden, wie bei der Augeninnendruckmessung, wasserlösliche Materialien benötigt, welche sich problemlos eintrocknen lassen, können diese als Tropfen auf die Oberseiten **5** und **6** aufgebracht und eingetrocknet werden. Durch die Tränenflüssigkeit, beim Anlegen an das Augenlid werden dann die beiden Stoffe herausgelöst und können ihre Wirkung im bzw. auf dem Auge entfalten.

Werden Materialien verwendet, deren Wirksamkeit durch ein Eintrocknen verändert wird, wird man auf der entsprechenden Oberseite **5** bzw. **7** einen Tropfen **11** aufbringen. Dieser Tropfen **11** wird dann, wie in **Figur 1** angedeutet und in **Figur 2** vergrössert dargestellt, mit einer Folie **13** dicht abgedeckt. Die Folie **13** ist kissenartig an ihren Rändern **15** fluiddicht mit der Stäbchenoberseite, hier der Seite **5** verbunden. Das aus der Folie **13** gebildete Kissen **16** hat an seinem dem Tägerende **17**, d.h. der Stirnseite **19** des Stäbchens **1** benachbarten Seite **20**, eine Aufreisslinie **21**. Durch einen mechanischen Druck auf das Kissen **16**, ausgeübt durch Daumen oder Zeigefinger, reisst dann das Kissen **16** entlang der Aufreisslinie **21** unter einem Austritt der im Kissen **16** enthaltenen Flüssigkeit auf. Bei der Augeninnendruckmessung wird man dann das Trägerende **17** in das Augenlid stecken und das Kissen **16** zum Aufreissen bringen, worauf die im Kissen **16** enthaltene Flüssigkeit **11** ins Augenlid eingebracht wird.

Anstatt den Tropfen mit einer Folie kissenartig zu überdecken und die Folie mit der Stäbchenoberfläche flüssigkeitsdicht zu verbinden, kann auch auf der Oberfläche des Endbereichs eine flüssigkeitsdichte Überdeckung angebracht werden, auf die dann der Tropfen aufgebracht wird, der dann mit der Folie kissenartige überdeckt wird.

Es kann aber auch gleich ein Material gefülltes Kissen auf der Oberfläche des Endbereichs befestigt (z.B. aufgeklebt) werden.

Zur Wahrung der Sterilität wird man den gesamten Träger in einer nicht dargestellten, aufreissbaren sterilen Verpackung unterbringen.

Anstatt die beiden Materialien auf der Vorder- und der Rückseite **5** und **6** am Stäbchenende **3** zu speichern, können die beiden Materialien auch, wie in Figur **3** gezeigt, in nebeneinander liegenden Bereichen **22a** und **22b** im Endbereich eines Stäbchens **26** angeordnet bzw. abgespeichert werden. **Figur 3** zeigt zwei, in den Bereichen **22a** und **22b** befindliche, eingetrocknete Flüssigkeitstropfchen **23** und **24**, welche dann zum Gebrauch durch vorhandenes Wasser bzw. Tränenflüssigkeit herausgelöst werden. Als Stäbchenmaterial kann ein Filterstreifen **25** verwendet werden, der derart steif ist, dass er beim Halten an seinem dem Materialauftragungsbereich **28** entgegengesetzten Ende **27** annähernd gerade bleibt. Die beiden Flüssigkeiten **23** und **24** werden auf den Filterstreifen **25** als Tropfen nebeneinander aufgebracht und anschliessend eingetrocknet. Unmittelbar nach dem Eintrocknen erfolgt, wie oben angetönt, die sterile Verpackung. Damit die beiden Tropfen durch die Fliesseigenschaft des Filterstreifens **25** nicht ineinander fliessen können, hat der Filterstreifen **25** zwischen den Orten für die beiden Flüssigkeitstropfen eine Verdichtungslinie **29**. Die Breite der Verdichtungslinie **29** als Zwischenraumbreite und die dortige Flieshemmung des verdichten Filtermaterials als Zwischenraummaterial für benachbarte Materialien wirkt als Flüssigkeitssperre, damit ein Zusammenfliessen benachbarter Materialien während des Aufbringens und einer anschliessenden Trocknungsphase vermieden wird. Damit sich die aufgebrachten Flüssigkeitstropfen nicht zu weit zum Trägerende **27** ausbreiten können, wodurch sich eine zu geringe Materialdichte des eingetrockneten Materials ergeben könnte, kann zum Ende **27** hin eine weitere als Flüssigkeitssperre dienende Verdichtungslinie **31** vorgesehen werden.

Die als Flüssigkeitssperre dienenden Verdichtungslinien **29** bzw. **31** können nun durch Einpressen eines Streifens erhalten werden. Es kann aber auch ein Sperrstoff beim Einpressen hinzugefügt werden. Der Sperrstoff sollte derart beschaffen sein, dass er in das Filtermaterial eindringt, die Poren und Zwischenräume im Filtermaterial verschliesst und durch Wasser sowie die aufzubringenden Materialien nicht mehr lösbar ist.

Der Sperrstoff kann auch ohne Verpressen aufgebracht werden.

Anstatt lediglich zwei Materialien nebeneinander oder auf benachbarten, aber auf entgegengesetzten Oberseiten aufzubringen, können mehr als nur diese beiden Materialien aufgebracht werden, in dem man mehrere Materialien nebeneinander und "Rücken an Rücken" aufbringt. Auch die mehr als zwei Materialien könnten kissenartig jedes für sich eingeschlossen werden.

Es kann aber auch eine gemischte Abspeicherung der Materialien erfolgen, wobei einige in Kissen eingeschlossen sind und die anderen eingetrocknet sind.

Auch kann man eingetrocknete Materialien am Stäbchenende zwischen einem Kissen mit Lösungsflüssigkeit und dem Stäbchenende abspeichern. Auch dieses Kissen hat dann eine Aufrisslinie. Die aus dem durch Fingerdruck aufgerissenen Kissen ausströmende Lösungsflüssigkeit schwemmt dann die vor ihr liegenden Materialien aus und transportiert sie z.B. bei einer Augeninnendruckmessung in das Augenlid, von wo aus sie dann übers Auge verteilt werden.

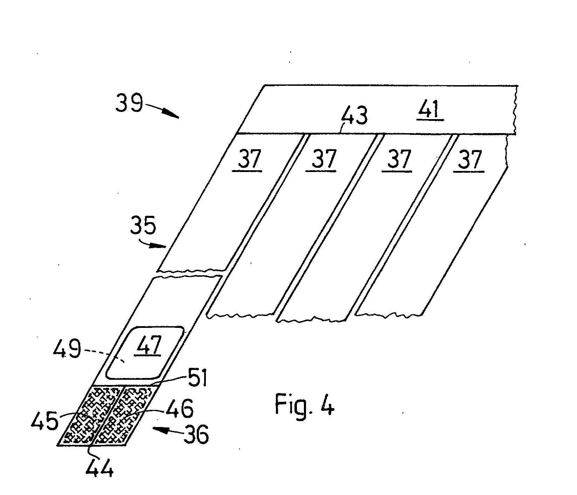

In der Regel wird man jeweils einen einzigen Träger steril verpacken. Es können aber auch mehrere Träger **35** zusammen steril verpackt werden. Eine Anordnung mit mehreren Träger **35** zeigt **Figur 4**. Die Träger **35** sind an ihrem dem Material tragenden Endbereich **36** entgegengesetzten Endbereich **37** nach Art eines Zündholzbriefchens **39** miteinander über ein Brückenelement **41** verbunden. Zum leichteren Abtrennen der einzelnen Träger **35** ist eine perforierte Trennlinie **43** zum Brückenelement **41** hin vorhanden. Anstelle einer perforierten Trennlinie **43** können andere Elemente verwendet werden, wie z.B. Materialverdünnungen, welche ein leichtes Abtrennen ermöglichen. Die hier gezeigten Träger **35** haben an ihrem freien Endbereich **36** zwei durch eine Flüssigkeitssperre **44** voneinander getrennte, eingetrocknete Materialien **45** und **46,** welche durch eine in einem Kissen **47** gespeicherte Flüssigkeit **49** auswasch- und zum Trägerende hin transportierbar sind. Das Kissen **47** hat, wie bereits oben beschrieben, hier aber nicht erkennbar, eine Aufreisslinie, welche durch Fingerdruck auf das Kissen dieses entlang der Aufreisslinie zum Bersten bringt. Zwischen Kissen **47** und den eingetrockneten Materialien **45** und **46** ist ebenfalls eine Flüssigkeitssperre **51** vorhanden.

Die Anordnung in "Briefchen" **39** kann auch bei steril zu haltenden Trägern vorgenommen werden; vorteilhafterweise wird man eine derartige Anordnung jedoch bei nicht steril zu handhabenden Trägern im Bereich der Mechanik und Kennzeichnung vornehmen.

## Patentansprüche

1. Material abgebender Träger, **dadurch gekennzeichnet, dass** der Träger wenigstens einen Bereich **(3/5; 22a)** für ein erstes Material **(7; 23; 45)** und wenigstens einen zweiten **(3/6; 22b)** vom ersten getrennten Bereich für ein zweites Material **(9; 24; 46)** aufweist.

2. Träger für Fluorescein als ein erstes Material **(7)**, insbesondere zur Verwendung in der Ophthalmologie, nach Anspruch 1, **dadurch gekennzeichnet, dass** der Träger getrennt von dem Fluorescein tragenden Bereich **(3/5; 22a)** einen weiteren Bereich **(3/6; 22b)** mit einem Anästhetikum als wenigstens ein zweites Material **(9)** aufweist.

3. Träger nach Anspruch 1 oder 2, **dadurch gekennzeichnet, dass** er stabförmig, insbesondere als Vierkantstab **(1 26)** ausgebildet ist und das erste und das wenigstens eine zweite Material **(7, 9; 23, 24; 45, 46)** in einem einzigen Stabendbereich **(3; 17; 36)** angeordnet sind.

4. Träger nach einem der Ansprüche 1 bis 3, **dadurch gekennzeichnet, dass** er als Filterpapierstreifen **(35)** ausgebildet ist, auf dem das erste und das wenigstens eine zweite Material **(45, 46),** vorzugsweise in wasserlöslicher Form, aufbringbar und trockenbar sind.

5. Träger nach einem der Ansprüche 1 bis 4, dadurch gekennzeichent, dass der das erste und der das wenigstens eine zweite Material **(23, 24; 45, 46)** tragende Bereich **(28; 36)** durch einen Zwischenraum **(29, 44)** voneinander getrennt sind, wobei die Zwischenraumbreite und die Flieshemmung des Zwischenraummaterials für benachbarte Materialien **(23, 24; 45, 46)** und eine Flüssigkeitssperre derart gewählt sind, dass ein Zusammenfliessen benachbarter Materialien **(23, 24; 45, 46)** als Flüssigkeit während des Aufbringens und/oder einer anschliessenden Trocknungsphase vermeidbar ist.

6. Träger nach einem der Ansprüche 1 bis 5, **dadurch gekennzeichnet, dass** wenigstens eines der Materialien **(11)** flüssig in einer kissenartigen Umhüllung **(16; 47)** angeordnet ist und jedes Kissen **(16; 47)** an seiner dem Trägerende **(17)** benachbarten Seite wenigstens eine Aufreisslinie **(21)** aufweist, welche auf einen mechanischen Druck auf das Kissen **(16; 47)** aufreissbar ist, damit der Kisseninhalt an einen vorgegebenen Ort bringbar, beispielsweise in das Augenlid einbringbar ist.

7. Träger nach einem der Ansprüche 1 bis 6, **dadurch gekennzeichnet, dass** mit einer sterilen, vorzugsweise aufreissbaren Verpackung umhüllt ist.

8. Trägeranordnung mit mehreren Trägern **(35)** nach einem der Ansprüche 1 bis 7, **dadurch gekennzeichnet, dass** die Träger **(35)** an ihrem dem Material tragenden Endbereich **(36)** entgegengesetzten Endbereich **(37)** nach Art eines Zündholzbriefchens miteinander verbunden, jedoch voneinander trennbar sind.
